# EUROPEAN PATENT APPLICATION

(11) **EP 4 670 640 A1**
(43) Date of publication of application: **31.12.2025**
(21) Application number: 25183287.9
(22) Date of filing: 17.06.2025
(51) Int. Cl.: A61B 6/42, A61B 6/00, A61B 6/51

(54) **A SOLUTION FOR DENTAL X-RAY IMAGING OF A PATIENT**

(30) Priority: 20.06.2024 FI 20245793
(71) Applicant: PaloDEx Group Oy, 04301 Tuusula (FI)
(72) Inventor: POLLARI, Mika, 01520 Vantaa (FI)
(74) Representative: Berggren Oy

(57) **Abstract**

The invention relates to a dental X-ray imaging system (100) for dental X-ray imaging of a patient (300). The system (100) comprises: a dental X-ray imaging unit (102) comprising: an X-ray source part (114), an X-ray imaging detector part (116) comprising one X-ray detector, and a gantry part (112) comprising the X-ray source part (114) and the X-ray imaging detector part (116); and a control system (106). The control system (106) is configured to: control the parts of the dental X-ray imaging unit (102) to acquire an X-ray scout image (302) of the patient (300) by using a first part of an active area of the X-ray detector, and control the parts of the dental X-ray imaging unit (102) to acquire panoramic dental X-ray image data of the patient (300) by using a second part of the active area of the X-ray detector being respective to the panoramic imaging. The first part of the active area is larger than the second part of the active area. The invention relates also to a method for a panoramic dental X-ray imaging of a patient (300), a computer program (714), and a tangible non-volatile computer-readable medium.

## Description

### TECHNICAL FIELD

The invention concerns in general the technical field of dental X-ray imaging. Especially the invention concerns panoramic dental X-ray imaging.

### BACKGROUND

Typically, the correct positioning of a patient may be one of the most time-consuming tasks of a user, e.g. an operator, of a dental X-ray imaging unit in a dental X-ray imaging process, but also one of the most important tasks. Especially, in panoramic imaging the correct positioning of the patient is important concerning the quality of the resulting dental X-ray image. Incorrect positioning of the patient may lead to a non-optimized image quality and/or additional X-ray imaging of the patient.

Traditionally, the patient may be positioned to the dental X-ray imaging unit using various supporting methods that are supposed to hold a head of the patient as stationary as possible. Traditional supporting means may be a chin rest, a static bite stick, and a head support, where the forehead, temple, and/or back of the skull is supported. In addition, different kind of straps may be used to make the patient positioning as rigid as possible. In addition, some dental X-ray imaging units have such bite sticks that are attached to the dental X-ray imaging unit such that attachment means allow movements of the bite sticks in some directions.

One approach that can be considered as traditional as well is using scout images. The scout images are a small dose projection images that can be used as a targeting aid for a panoramic image.

Furthermore, typically in the panoramic imaging anatomic shapes of the patient are unknown prior taking the panoramic image. Panoramic image quality is affected heavily based on how well a pre-defined imaging layer corresponds with the actual anatomic shapes, e.g. dental arch, of the patient. Typically, an average shape is used for all patients, which may lead to a non-optimized image quality.

### SUMMARY

The following presents a simplified summary in order to provide basic understanding of some aspects of various invention embodiments. The summary is not an extensive overview of the invention. It is neither intended to identify key or critical elements of the invention nor to delineate the scope of the invention. The following summary merely presents some concepts of the invention in a simplified form as a prelude to a more detailed description of exemplifying embodiments of the invention.

An objective of the invention is to present a dental X-ray imaging system, a method, a computer program, and a computer-readable medium for dental X-ray imaging of a patient. Another objective of the invention is that the dental X-ray imaging system, the method, the computer program, and the computer-readable medium for dental X-ray imaging of a patient improve quality of panoramic dental X-ray images.

The objectives of the invention are reached by a dental X-ray imaging system, a method, a computer program, and a computer-readable medium as defined by the respective independent claims.

According to a first aspect, a dental X-ray imaging system for dental X-ray imaging of a patient is provided, wherein the system comprises: a dental X-ray imaging unit comprising: an X-ray source part for emitting an X-ray beam, an X-ray imaging detector part comprising one X-ray detector for receiving the X-ray beam from the X-ray source part, and a gantry part comprising the X-ray source part and the X-ray imaging detector part; and a control system configured to: control the parts of the dental X-ray imaging unit to acquire an X-ray scout image of the patient by using a first part of an active area of the X-ray detector, and control the parts of the dental X-ray imaging unit to acquire panoramic dental X-ray image data of the patient by using a second part of the active area of the X-ray detector being respective to the panoramic imaging, wherein the first part of the active area is larger than the second part of the active area.

The first part of the active area of the X-ray detector may comprise the entire active area of the X-ray detector.

The control of the parts of the dental X-ray imaging unit to acquire the X-ray scout image may comprise controlling a collimator of the X-ray source part to collimate the X-ray beam into a cone beam.

Alternatively or in addition, the control of the parts of the dental X-ray imaging unit to acquire the panoramic dental X-ray image data may comprise controlling the collimator of the X-ray source part to collimate the X-ray beam into a narrow beam.

The control system may be configured to determine dental arch data of the patient based on the X-ray scout image.

The control system may further be configured to control the parts of the dental X-ray imaging unit to acquire the panoramic dental X-ray image data of the patient according to the determined dental arch data.

The determining the dental arch data may comprise that the control system is configured to: detect a plurality of anatomical structures of the patient from the X-ray scout image, determine positions of the plurality of anatomical structures of the patient, and determine dental arch data of the patient based on the determined positions of the detected plurality of anatomical structures of the patient.

The control system may be configured to apply at least one trained detection model to detect the plurality of anatomical structures of the patient from the X-ray scout image.

The at least one trained detection model may be a machine learning (ML) model or an artificial intelligence (AI) model.

The control system may be configured to determine the positions of the plurality of anatomical structures of the patient by utilizing atlas data.

The control system may further be configured to: detect at least one patient position error based on the plurality of anatomical structures of the patient, and determine patient position correction data for correcting the at least one patient position error.

The control system may be configured to perform at least one of the following: use the patient position correction data in the controlling of the parts of the dental X-ray imaging unit to acquire the panoramic dental X-ray image data of the patient, generate a guidance to the patient and/or to an operator of the dental X-ray imaging system based on the patient position correction data, use the patient position correction data to minimize the effect of at least one patient position error.

According to a second aspect, a method for a panoramic dental X-ray imaging of a patient is provided, wherein the method is performed by an X-ray dental imaging system as described above, wherein the method comprises: controlling the parts of the dental X-ray imaging unit to acquire an X-ray scout image of the patient by using a first part of an active area of the X-ray detector, and controlling the parts of the dental X-ray imaging unit to acquire panoramic dental X-ray image data of the patient by using a second part of the active area of the X-ray detector being respective to the panoramic imaging, wherein the first part of the active area is larger than the second part of the active area.

According to a third aspect, a computer program is provided, wherein the computer program comprises instructions which, when the program is executed by an X-ray dental imaging system as described above, cause the X-ray dental imaging system to carry out the method as described above.

According to a fourth aspect, a tangible non-volatile computer-readable medium is provided, wherein the tangible non-volatile computer-readable medium comprises instructions which, when executed by an X-ray dental imaging system as described above, cause the X-ray dental imaging system to carry out the method as described above.

Various exemplifying and non-limiting embodiments of the invention both as to constructions and to methods of operation, together with additional objects and advantages thereof, will be best understood from the following description of specific exemplifying and non-limiting embodiments when read in connection with the accompanying drawings.

The verbs "to comprise" and "to include" are used in this document as open limitations that neither exclude nor require the existence of unrecited features. The features recited in dependent claims are mutually freely combinable unless otherwise explicitly stated. Furthermore, it is to be understood that the use of "a" or "an", i.e. a singular form, throughout this document does not exclude a plurality.

### BRIEF DESCRIPTION OF FIGURES

The embodiments of the invention are illustrated by way of example, and not by way of limitation, in the figures of the accompanying drawings.
Figure 1 illustrates schematically an example of a dental X-ray imaging system for dental X-ray imaging of a patient.
Figure 2 illustrates schematically an example of a method for dental X-ray imaging of a patient.
Figure 3A illustrates schematically an example of lateral (LAT) imaging of a patient for acquiring an X-ray scout image of the patient.
Figure 3B illustrates schematically an example of a LAT X-ray scout image of a patient.
Figure 3C illustrates schematically a non-limiting example of a first part of an active area of an X-ray detector used for acquisition of an X-ray scout image.
Figure 3D illustrates schematically a non-limiting example of a second part of an active area of an X-ray detector used for acquisition of panoramic dental X-ray image data.
Figure 4 illustrates schematically an example of method steps of determining dental arch data.
Figure 5 illustrates schematically an example of a non-limiting example of a plurality of landmarks intended for detecting a plurality of anatomical structures of a patient from an X-ray scout image.
Figure 6 illustrates schematically an example of method steps of correcting at least one patient position error.
Figure 7 illustrates schematically an example of a control system of a dental X-ray imaging system.

### DESCRIPTION OF THE EXEMPLIFYING EMBODIMENTS

In this description we use the following vocabulary concerning different phases of a dental X-ray imaging process. The term radiating means the phase comprising merely the irradiation, i.e. the phase when an X-ray source is providing an X-ray beam that travels through an object to an X-ray imaging detector. The object may be expected to remain as still, i.e. immobile, as possible during the radiating. During the radiating one or more parts of the dental X-ray imaging unit may move. The term scanning, in turn, means the phase comprising the radiating and moving of one or more parts of the dental X-ray imaging unit. The scanning does not comprise positioning of one or more parts of the X-ray imaging unit in a correct place for providing X-ray images. The term imaging means the whole process comprising radiating, scanning and positioning.

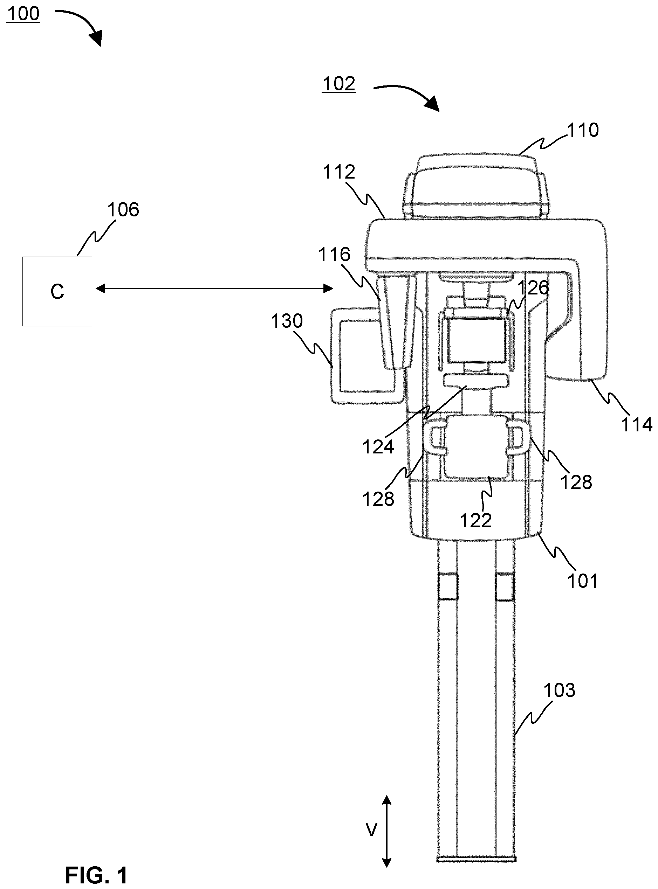

Figure 1 illustrates an example of a dental X-ray imaging system 100 for dental X-ray imaging of an object (for sake of clarity the object is not shown in Figure 1). The imaging system 100 comprises a dental X-ray imaging unit 102 for acquiring X-ray image data from the object, e.g. a patient 300 or calibration target, in dental X-ray imaging, e.g. in extraoral dental X-ray imaging. The acquired X-ray image data is used to form a two-dimensional (2D) X-ray image or to reconstruct a three-dimensional (3D) X-ray volume from at least part of imaged object. The dental X-ray imaging system 100 further comprises a control system 106. The control system 106 may be electrically and/or communicative-ly coupled to the dental X-ray imaging unit 102. The implementation of the control system 106 may be done as a stand-alone unit or as a distributed control environment between a plurality of stand-alone units providing distributed controlling resource. Preferably, the control system 106 may be an embedded computer. The control system 106 may for example comprise a control unit of the dental X-ray imaging unit 102. The control system 106 may further comprise a computing unit being external to the dental X-ray imaging unit 102 (e.g. a cloud computing unit). The control unit of the dental X-ray imaging unit 102 is configured to control the operation of the dental X-ray imaging unit 102 at least in part. The control unit of the dental X-ray imaging unit 102 may be located proximate to the dental X-ray imaging unit 102 or the control unit of the dental X-ray imaging unit 102 may be embedded withing the dental X-ray imaging unit 102. The X-ray imaging unit 102 may further comprise one or more user interfaces 130, e.g. display(s), screen(s), and/or touch screen(s).

The dental X-ray imaging unit 102 may be configured to perform different types of imaging procedures (i.e. imaging modes), including, but not limited to computed tomography (CT) imaging and/or panoramic imaging. The CT imaging may be a cone beam CT (CBCT) imaging or any other type of CT imaging. The CT imaging results (i.e. produces) the X-ray image data for the reconstruction (i.e. formation) of 3D volume from the at least part of the imaged object. The panoramic imaging may for example be standard panoramic imaging, pediatric panoramic imaging, orthozone panoramic imaging, wide arch panoramic imaging, orthogonal panoramic imaging or the like. The panoramic imaging results the X-ray image data for the reconstruction of panoramic 2D image. Alternatively or in addition, the dental X-ray imaging unit 102 may be configured to perform cephalometric imaging, if the dental X-ray imaging unit 102 is equipped with parts, which are necessary for the cephalometric imaging. The cephalometric imaging may for example be cephalo pediatric lateral projection, cephalo lateral projection, cephalo posterior-anterior, and/or the like. The cephalometric imaging results the X-ray image data for the formation of cephalometric 2D image. Figure 1 illustrates only one example of a dental X-ray imaging unit 102 for use with the concepts in the present disclosure.

The dental X-ray imaging unit 102 comprises a carriage part 101 that may be moveably supported on a supporting column 103. The carriage part 101 may be moved up and down in a height direction, i.e. the vertical direction (V), by means of a guide motor (not shown in Figure 1) that is configured to move the carriage part 101 up and down along the supporting column 103 in the height direction. An upper shelf, 110 is configured to support a gantry part, i.e. a rotating part, 112, which is rotatable in a horizontal plane with respect to the upper shelf 110. The upper shelf 110 and/or gantry part 112 may comprise a rotating motor (not shown in Figure 1) configured to rotate the gantry part 112. Alternatively or in addition, the upper shelf 110 may comprise a pivot motor (not shown in Figure 1) configured to pivot the upper shelf 110 around the supporting column 103. The upper shelf 110 and/or the gantry part 112 may alternatively or in addition comprise at least one linear motor (not shown in Figure 1) configured to provide linear movement(s). Alternatively or in addition, the dental X-ray imaging unit 102 may be mounted to a supporting structure (not shown in Figure 1) exemplarily a wall to being supported by the supporting column 103.

The dental X-ray imaging unit 102 comprises further an X-ray source part 114 and an X-ray imaging detector part 116, which are used in the acquisition of the X-ray image data. The gantry part 112 embodies and supports the source part 114 and the imaging detector part 116. The gantry part 112 may have substantially a form of letter C, as presented in Figure 1, whereupon the source part 114 may be attached on one end of the gantry part 112 and the imaging detector part 116 may be attached on the other end of the gantry part 112 so that the source part 114 and the imaging detector part 116 are opposed from each other. The X-ray source part 114 comprises an X-ray source that emits X-rays (i.e. generates the X-ray beam) through the object being imaged, e.g. a head of the patient 300, to the X-ray imaging detector part 116, which comprises one X-ray detector that receives the emitted X-rays from the source part 114. The X-ray imaging detector part 116 further generates the X-ray image data from the X-ray exposed, i.e. imaged, object.

The dental X-ray imaging unit 102 also comprises a collimator (not shown in Figure 1) for the X-ray source part 114 to restrict and/or shape the beam of X-rays. The X-rays pass through a portion of the object, for example the patient's anatomy, e.g. patient's head. The anatomical structures through which the X-rays pass may absorb varying amounts of the X-ray energy. After passing through the object, the attenuated X-rays are received by the X-ray imaging detector part 116. The X-ray imaging detector part 116 is configured to convert the magnitude of the received X-ray energy and to produce a digitized output, i.e. the X-ray image data, representative of the unabsorbed X-rays at the at least one X-ray detector. The collection of digitized outputs from the X-ray imaging detector part 116 that correspond to a single emission of a beam of X-rays from the X-ray source part 114 may be referred to a projection image of the object being imaged, for example the head of the patient.

Furthermore, the dental X-ray imaging unit 102 may comprise patient support parts 124, 126 (as presented in Figure 1, but not necessarily) that may be used for supporting the patient 300 in the CT or panoramic imaging. The patient support parts 124, 126 may comprise a chin support part 124 and/or a head support part 126. The chin support part 124 may support a tip of a chin of patient and the head support part 126 may support a forehead or temple of the patient. The dental X-ray imaging unit 102 may comprise a lower shelf 122 that extends from the carriage part 101. The lower shelf 122 may comprise the chin support part 124 as in the example dental X-ray imaging unit 102 of Figure 1. The head support part 126 may extend from the upper shelf 110 through the gantry part 112 as in the example dental X-ray imaging unit 102 of Figure 1. Alternatively, the lower shelf 122 may also comprise the head support part 126. The patient support parts, i.e. the chin support part 124 and/or the head support part 126, may be optional, and positioning of the patient 300 may be carried out in other manners. The dental X-ray imaging unit 102 may further comprise handles 128 for the patient to grasp. The dental X-ray imaging unit 102 may further comprise a bite block, e.g. a bite stick, (not shown in Figure 1) arranged for example to the chin support part 124.

The gantry part 112 may be rotated by the rotating motor, for example. The rotation of the gantry part 112 rotates the X-ray source part 114 and the X-ray imaging detector part 116 around the object to be imaged, for example around a rotation axis along a motion path. As the X-ray source part 114 and the X-ray imaging detector part 116 are rotated around the object, for example the head of the patient 300, the X-ray imaging device 102 operates to acquire a plurality of projection images of the object taken at incremental angles of rotation. The dental X-ray image may be formed from the plurality of projection images by reconstructing the X-ray image data to the dental X-ray image.

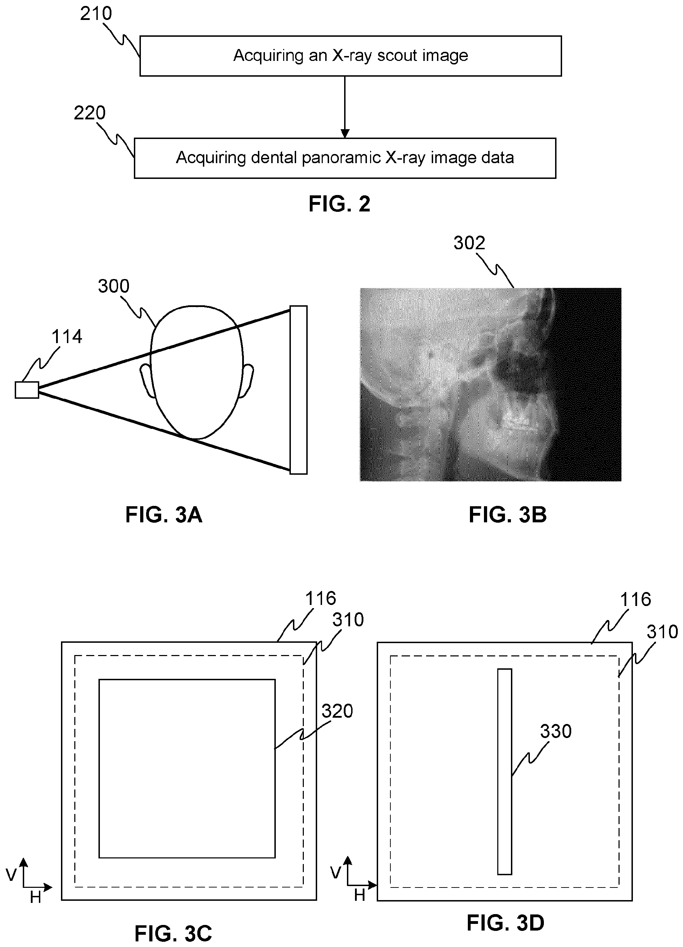

Next at least some example aspects of a method for panoramic dental X-ray imaging of an object, e.g. a patient 300, are defined referring to Figure 2. Figure 2 illustrates the method as a flow chart. The method is performed by the dental X-ray system 100 discussed above.

At step 210, the control system 106 controls the parts of the dental X-ray imaging unit 102 to acquire an X-ray scout image 302 of the patient 300 by using a first part 320 of an active area 310 of the X-ray detector of the X-ray imaging detector part 116. Typically, the active area 310 of the X-ray detector has a square shape or a rectangular shape. The first part 320 of the active area 310 of the X-ray detector may comprise the entire active area 310 of the X-ray detector. This enables as large size of the X-ray scout image 302 as possible. Alternatively, the first part 320 of the active area 310 may comprise only a part of the active area 310 of the X-ray detector. The size of the first part 320 of the active area 310 used for the acquisition of the X-ray scout image 302 may vary depending on the desired size of the X-ray scout image 302. The larger the size of the X-ray scout image 302 is, the wider the area, from which anatomical structures of the patient 300 may be detected from the X-ray scout image 302, is. The controlling of the parts of the dental X-ray imaging unit 102 by the control system 106 to acquire the X-ray scout image 302 may comprise controlling the collimator of the X-ray source part 114 to collimate (i.e. restrict and/or shape) the X-ray beam into a cone beam. In other words, the controlling of the parts of the dental X-ray imaging unit 102 by the control system 106 to acquire the X-ray scout image 302 may comprise controlling the collimator of the X-ray source part 114 so that the cone beam is collimated on the first part 320 of the active area 310 of the X-ray detector of the X-ray imaging detector part 116. The cone beam may for example have a cone shape or a pyramidal shape. The X-ray scout image 302 is a two-dimensional (2D) X-ray projection image. Preferably, the X-ray scout image 302 may be a lateral (LAT) X-ray scout image, i.e. a lateral view angle X-ray scout image of the patient. Figure 3A illustrates schematically an example of the lateral imaging of the patient 300 for acquiring the X-ray scout image 302 of the patient 300. Figure 3B illustrates schematically an example of the X-ray scout image 302 of the patient 300 acquired by the lateral imaging. The example X-ray scout image 302 of Figure 3B is a LAT X-ray scout image. Figure 3C illustrates schematically a non-limiting example of the first part 320 of the active area 310 of the X-ray detector used for the acquisition of the X-ray scout image 302. In the example of Figure 3C the first part 320 of the active area 310 comprise a part of the active area 310 of the X-ray detector. However, the first part 320 of the active area 310 may also comprise the entire active area 310 of the X-ray detector as discussed above.

At step 220, the control system 106 controls the parts of the dental X-ray imaging unit 102 to acquire panoramic dental X-ray image data of the patient 300 by using a second part 330 of the active area 310 of the X-ray detector of the X-ray imaging detector part 116 being respective to the panoramic imaging. In other words, the control system 106 controls the parts of the dental X-ray imaging unit 102 to acquire the panoramic dental X-ray image data by using the same X-ray detector of the X-ray imaging detector part 116 that was used for acquiring the X-ray scout image 302 at the step 210. The first part 320 of the active area 310 of the X-ray detector is larger than the second part 330 of the active area 310 of the X-ray detector. In other words, the active area of the X-ray detector (i.e. the first part 320 of the active area 310) used for the acquisition of the X-ray scout image 302 is larger than the active area of the X-ray detector (i.e. the second part 330 of the active area 310) used for the acquisition of the panoramic dental X-ray image data. As the second part 330 of the active area 310 of the X-ray detector is respective to the panoramic imaging, the second part 330 of the active area 310 of the X-ray detector comprises a narrow linear area. The narrow linear area is preferably a narrow vertical linear area. In other words, the horizontal dimension of the second part 330 of the active area 310 is substantially smaller in relation the vertical dimension of the second part 330 of the active area 310. The controlling of the parts of the dental X-ray imaging unit 102 to acquire the panoramic dental X-ray image data may comprise controlling the collimator of the X-ray source part 114 to collimate (i.e. restrict and/or shape) the X-ray beam into a narrow beam. The narrow beam is preferably a narrow vertical beam. In other words, the controlling of the parts of the dental X-ray imaging unit 102 to acquire the panoramic dental X-ray image data may comprise controlling the collimator of the X-ray source part 114 so that the narrow beam is collimated on the second part 330 of the active area 310 of the X-ray detector of the X-ray imaging detector part 116. Figure 3D illustrates schematically a non-limiting example of the second part 330 of the active area 310 of the X-ray detector used for the acquisition of the panoramic dental X-ray image data.

The use of the first part 320 of the active area 310 of the X-ray detector (being larger than the active area of the X-ray detector used for the acquisition of the panoramic dental x-ray image data) for the acquisition of the X-ray scout image 302 enables acquisition of a single large X-ray scout image 302 to be used in the panoramic dental X-ray imaging. Especially, when the first part 320 of the active area 310 comprises the entire active area 310 of the X-ray detector, the size of the X-ray scout image 302 may be maximized. Typically, in the panoramic dental X-ray imaging multiple X-ray scout images are acquired by using the active area of the X-ray detector respective to the panoramic imaging or by using a separate panoramic X-ray detector, which is a narrow linear detector having a narrow linear active area.

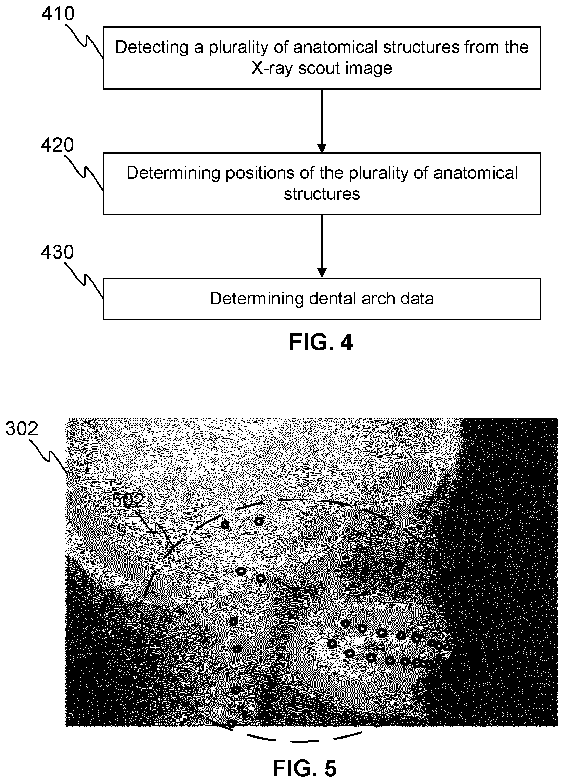

The control system 106 may determine dental arch data of the patient 300 based on the X-ray scout image acquired at the step 210. Figure 4 illustrates schematically an example of method steps of determining the dental arch data based on the X-ray scout image 302.

At step 410, the control system 106 may detect a plurality of anatomical structures of the patient 300 from the X-ray scout image 302. The control system 106 may detect the plurality of anatomical structures of the patient from the X-ray scout image 302 by using automated detection. The automated detection comprises applying at least one trained detection model 716 to the X-ray scout image 302. In other words, the control system 106 may detect the plurality of anatomical structures of the patient from the X-ray scout image 302 by apply at least one trained detection model 716. The X-ray scout image 302 is used as the input data of the at least one trained detection model 716 and the plurality of anatomical structures of the patient is obtained as the output data of the at least one trained detection model 716. The detection of the plurality anatomical structures of the patient 300 means that the plurality of anatomical structures is localized i.e. their spatial location in the X-ray scout image 302 is defined, and their identity (e.g. name of label) is defined.

The at least one trained detection model 716 may be formed by using training data. The training of the at least one detection model 716 may preferably be supervised training, i.e. supervised learning, but also other training paradigms may be used, e.g. unsupervised learning, reinforcement learning, or hybrid paradigms comprising two or more training paradigms. In the supervised training, the training data comprises input training data and output training data. The input training data may for example comprise a plurality of X-ray scout images. The plurality of X-ray scout images comprised in the input training data may for example comprise previously collected X-ray scout images. The previously collected X-ray scout images may preferably comprise X-ray scout images of patients. Alternatively or in addition, the previously collected X-ray scout images may comprise X-ray scout images of phantoms (e.g. dry skulls). Alternatively or in addition, the plurality of X-ray scout images comprised in the input training data may comprise simulated and/or artificial X-ray scout images. Data augmentation may also be used to produce further input training data. The output training data may for example comprise annotation data representing annotations of the anatomical structures, e.g. the positions and the identities of the anatomical structures. The output training data may be generated in a manual process, e.g. by a human expert, in a semi-automated process, or in a fully automated process. The at least one trained detection model 716 may be based on one or more machine learning (ML) methods or one or more artificial intelligence (AI) methods. In other words, the at least one trained detection model 716 may be a ML model or an AI model. For example, the at least one trained detection model 716 may be based on a tree-based machine learning (ML) methods, e.g. decisions trees, and especially regression trees. Preferably, the at least one trained detection model 716 may be based on ensemble of regression trees ML method. Alternatively, the at least one trained detection model 716 may be based on convolutional neural networks (CNNs).

The at least one trained detection model 716 may be stored into a memory part of the control system 106.

The detecting of the plurality of anatomical structures of the patient 300 from the X-ray scout image 302 may for example be based on landmark -based detection. In the landmark -based detection a plurality of landmarks is detected from the X-ray scout image 302 by using the automated detection, i.e. by applying the at least one trained detection model 716. In other words, the X-ray scout image 302 may be used as the input data of the at least one trained detection model 716 and the plurality of landmarks may be obtained as the output data of the at least one trained detection model 716. The plurality of landmarks represents the plurality of anatomical structure of the patient 300. Because the X-ray scout image 302 is 2D X-ray image, the detected plurality of landmarks are 2D-landmarks. The plurality of landmarks may comprise any relevant anatomical structures from a head-neck region of the patient 300. Some non-limiting examples of the plurality of landmarks may comprise teeth (e.g. mandibular and/or maxillary teeth), temporomandibular joint(s) (TMJ(s)), one or more mandibular structures (e.g. coronoid(s), condyle(s), chin menton, and/or any other mandibular structures), one or more eye socket structures (e.g. lower orbit(s) and/or any other eye socket structures), auditory canal(s), one or more nose and/or skull structures (e.g. anterior nasal spine (ANS), posterior nasal spine (PNS) and/or any other nose and/or skull structures), one or more neck structures (e.g. upper cervical vertebras and/or any other neck structures), and/or any other anatomically relevant structures. Similarly, the detected plurality of anatomical structures of the patient 300 may comprise any relevant anatomical structures from the head-neck region of the patient 300. Thus, the above list of non-limiting examples of the plurality of landmarks applies also to the plurality of anatomical structures of the patient 300. The plurality of anatomical structures of the patient 300 to be detected from the X-ray scout image 302 may be selected depending on which part(s) of the head-neck region of the patient 300 is relevant in the imaging process in question. For example, for determining dental arch data of the patient 300 at least a plurality of dental arch related anatomical structures may be detected from the X-ray scout image 302. Figure 5 illustrates schematically a non-limiting example of the plurality of landmarks 502 intended for detecting the plurality of anatomical structures of the patient 300 from the X-ray scout image 302. In the example of Figure 5 the small circles inside the dashed ellipse represent the landmarks 502.

The control system 106 may further detect at the step 410 one or more other structures from the X-ray scout image 302. The one or more other structures may for example comprise at least one of the following: one or more foreign body structures, one or more soft tissue structures. The foreign body structures are non-anatomical objects origin from an external environment. Typically, foreign body structures made from metal and other radiopaque material may cause larger artefacts in the X-ray images than foreign bodies made from more radio translucent material. In addition to the material also the size and location of the foreign body structure may have an impact how severe the induced artefact is from the clinical point of view. One especially harmful example of the foreign body structure is a collar of an X-ray protection apron. If the collar of the X-ray protection apron is located too high, the collar will cast a large artefact (called as a shark fin artefact) that may shadow for example mandible teeth and even maxillary structures. Other common examples of the foreign body structures in the head-neck region of the patient 300 may comprise jewelries (e.g. necklace, earrings, metal piercings etc.), hair accessories (e.g. clips, hair pins, etc.), and/or eyeglasses, etc. The soft tissue structures may also cause artefacts in the X-ray images. For example, although patient's 300 head, neck and mandibular pose may be correct, the soft tissue of the patient 300 may still be incorrectly deformed. One common and harmful example of the artefacts caused by the soft issue structures is a misplaced tongue, where tongue is not pressed against the palate and there is an air cap in an oral cavity above the tongue. Other examples of the soft tissue structures that may cause artefacts in the X-ray images may comprise open mouth and/or lips.

The control system 106 may detect the one or more other structures from the X-ray scout image 302 by using the automated detection as described above, wherein the automated detection comprises applying at least one trained detection model 716 to the X-ray scout image 302. In other words, the control system 106 may detect the one or more other structures from the X-ray scout image 302 by applying the at least one trained detection model 716. The same at least one trained detection model 716 applied for detecting the plurality of anatomical structures of the patient 300 may also be applied for detecting the one or more other structures from the scout image 302. Also, the landmark - based detection used for detecting the plurality of anatomical structures from the X-ray scout image 302 may be used for detecting the one or more other structures from the X-ray scout image 302. Alternatively, specific at least one trained detection model may be applied to the X-ray scout image 302 to detect the one or more other structures. The detection of the one or more other structures from the X-ray scout image 302 by applying the specific at least one trained detection model may also comprise determining the positions of the detected one or more other structures. The detection of the one or more other structures by applying the specific at least one trained detection model may for example comprise at least one of the following tasks: a classification task, a detection task, a segmentation task. In the classification task the specific at least one trained detection model is trained for classifying which other structures, if any, the scout image 302 includes. In the detection task the specific at least one trained detection model is trained for locating the one or more other structures (e.g. by using bounding box) in the scout image 302. In the segmentation the specific at least one trained detection model is trained for portioning the scout image 302 into segments and identifying what kind of structure(s) is associated with the segments. The formulation may be binary, multi-class, or multi-label formulation. The specific at least one trained detection model may for example be based on one or more machine learning (ML) methods or one or more artificial intelligence (AI) methods. In other words, the specific at least one trained detection model may be a ML model or an AI model. For example, the specific at least one trained detection model 716 may be based on neural networks, deep neural networks, convolutional neural networks, recurrent neural networks, (mask) region-based convolutional neural networks, fast/faster R-CNN, residual neural networks, ResNet, You Only Look Once (YOLO), Single shot detectors, U-Net, transformers, Vision transformer, Histogram of oriented gradients (HoG), support vector machines, bag of features, decision trees, bagged decision trees, and/or random forest etc. Similarly, as the at least one trained detection model 716 described above, the specific at least one trained detection model may be formed by using the training data. The specific at least one trained detection model may be stored into the memory part of the control system 106.

Alternatively, the automated detection may comprise applying an image analysis and processing method for detecting the one or more other structures from the X-ray scout image 302. The most severe structures are presented as either high intensity / hyperdense (e.g. the shark fin) or low opacity / hypodense (e.g. the air cap due to the tongue drop). The positions of the one or more other structures may also be defined by applying the image analysis and processing method. The high intensity region related to the shark fin should be located at the lower border of the X-ray scout image 302 and behind the neck of the patient 300, while the low intensity region related to air-cap should be located upper part of the oral cavity of the patient 300. Thus, detecting and/or segmenting these one or more other structures is also possible using solely the image processing method. Futhremore, different hybrid combinations may also be applied to detect the one or more other structures. For example, the landmark information from the landmark -based detection may be used to extract relevant region of interest before applying the image analysis method and/or using the at least one trained detection model 716 for finetuning the results of the image analysis.

At step 420, the control system 106 may determine positions of the plurality of anatomical structures of the patient detected at the step 410. The control system 106 may determine the positions of the plurality of anatomical structures by using atlas data 718. As the plurality of anatomical structures of the patient 300 detected at the step 410 is presented in 2D image coordinates, the detected plurality of anatomical structures may be transformed into 3D imaging device coordinates (i.e. in the coordinates of the dental X-ray imaging unit 102). In other words, the control system 106 may determine the positions of the plurality of anatomical structure of the patient 300 in the 3D imaging device coordinates by using the atlas data 718.

The positions of the plurality of anatomical structures of the patient in the 3D imaging device coordinates may for example be used for correcting position errors of the patient 300. The goal is to align the atlas data 718 with the patient 300. If the atlas data 718 is aligned with patient 300 closely enough, the positions of the plurality of anatomical structures of the patient 300 in the 3D imaging device coordinates may directly be defined from the aligned atlas data. As the direct alignment between the patient 300 and the atlas data 718 is not possible, the problem is turned as an alignment of real 2D scout image extracted anatomical data of the patient 300 and virtual projection of similar anatomy data of the atlas data 718 using the same imaging geometry as used during the acquiring of the scout image 302. In other words, the alignment of the atlas data 718 with the patient 300 may be performed by using imaging geometry data representing an imaging geometry of the dental X-ray imaging unit 102 respective to the X-ray scout image 302. The control system 106 may for example obtain the imaging geometry data when the X-ray scout image 302 are acquired, i.e. in connection with the acquirement of the X-ray scout image 302, at the step 210. The goal of the process is to geometrically transform the atlas data 718 until its' virtually project anatomical data and the scout extracted anatomi-cal data are as similar as possible. The output is the aligned atlas data.

Main operations during the alignment may for example comprise: a transformation, a virtual projection, a cost function, a minimization, and a final alignment. The transformation modifies geometrical information of the atlas data 718. It may modify the position of geometrical information (such as locations of the anatomical structures) included the atlas data 718. These main operations during the alignment are discussed next more.

The transformation may modify the position of the anatomical structures and it may for example be a rigid body (6D) transformation. The transformation may also for example be a similarity transform (7D) transformation, which modifies both the position and the scale of the geometrical information comprised in the atlas data 718. The transformation may also comprise for example the rigid body transformation and an anisotropic scaling (9D) and thus allow also modification of the position, the scale, and the shape of the geometrical information comprised in the atlas data 718. The transformation may also for example be an affine (12D) that is even more flexible than 9D model. The above transformations are only some non-limiting examples of linear transformations that may be used in the transformation. Alternatively or in addition to the linear transformation, the transformation may be a non-linear transformation. Non-linear transformations typically have much higher number of free parameters and thus they allow very flexible shape deformations in addition to positional changes. In some cases, the transformation may be parametrized as a part of the atlas data 718 (e.g. statistical models/atlases may comprise shape modes). The transformations are well known and there are many other transformations that may also be used.

As the imaging geometry used in the acquiring the scout image 302 and the properties of the X-ray detector are known, one may virtually project transformed atlas data 718 (such as location of the anatomical structures) into the same imaging plane as the X-ray scout image 302. In a simple example a line going through an X-ray source (location known from the imaging geometry data) and the transformed anatomical structure position (known from the transformed atlas data) may be formed. Similarly, a virtual imagine plane presenting the X-ray detector may be formed by using the imaging geometry data and the X-ray detector properties. The intersection of the line and the plane in plane is the virtual projection of the anatomical structure in the virtual scout image plane. Repeating the process for all anatomical structures that will be used in the registration process two data in same scout image coordinate system may be defined: target data representing the positions of the anatomical structures extracted from the real X-ray scout image 302 and floating data representing virtually projected versions of the same structures. In each iteration of the registration process the virtually projected data may change as the transformation applied to the atlas data 718 changes whereas the target data remains fixed during the whole registration process.

The cost function determines the goodness of the registration. The smaller the cost is the better the registration is. According to a simple example, the anatomical data may be encoded as points, then after the virtual projection there are two anatomical point sets both defined in the imaging device coordinates. The cost function may for example be a distance-based cost function. In simple case the cost function may for example be the mean of all point pairs related to the same anatomical structure. Alternative or in addition to the arithmetic mean the cost function may also be a weighted mean, where different structures are weighted differently. The cost function may also be any other cost functions. In some cases, constrains may be given geometric transformation to prevent some (typically non-physical) transformations. Thus, in addition to the data dependent term, the cost function may also contain penalty term in order to regularize the transformation.

The registration is a minimization problem. Once the cost function is minimized with respect the transformation parameters, then the data sets are geometrically aligned. Typically, gradient-based minimization algorithms are used in minimization for simpler (linear) transformation, but also non-gradient based (powel, simplex etc.) minimization methods may be used. Minimization ends when at least one stopping criteria is fulfilled. The stopping criteria may for example be related to the cost-function value (i.e. the value drops under the stopping limit) or to the change rate (the cost function and/or the transformation parameter change between iterations is smaller than a set threshold) or something as simple as number of iterations exceeds the maximum iteration number. Minimization and stopping criteria are well known in optimization and only some non-limiting simple examples are given above, but there are many other possibilities that may also be used.

The ultimate goal of the alignment is to align the atlas data 718 with the patient 300 as discussed above. Here the 2D alignment of the scout extracted anatomical data and the virtually projected anatomical data from the atlas data 718 are used as proxies for the actual goal. Once the minimization convergences and stops to global minimum, then the anatomical structures of the patient and the anatomical structures of the atlas data 718 are aligned in the imaging device coordinates. In other words, the positions of the plurality of anatomical structures of the patient 300 are defined in the 3D imaging device coordinates by using the atlas data 718. Thus, the optimal transformation (end result of the minimization) is used to transform the atlas data 718. It should be noted that the atlas data 718 may include also other geometrical information than the anatomical information that is used in the registration. When the atlas data 718 is aligned with the patient 300 all geometrical instances (anatomical data, imaging related data etc.) are all transformed with the same optimal transformation. As a result, any geometrical information included in the atlas data 718 is aligned with the patient 300 in the imaging device coordinates.

The atlas data 718 may comprise atlas anatomy data. The atlas anatomy data of the atlas data 718 may for example comprise atlas anatomic structure data, i.e. information on at least one anatomical structure on the head-neck region. The atlas anatomic structure data may for example comprise at least a location of one or more anatomic structures. The atlas anatomy data may also comprise other information related to the one or more anatomical structures, e.g. label or identity of the anatomical structure(s). The atlas anatomy data may comprise any relevant anatomical structures from the head-neck region. For example, the atlas anatomy data may comprise, but is not limited to, one or more of the following anatomic structures: teeth (e.g. maxillary teeth and/or mandibular teeth), the TMJ(s), one or more mandibular locations (e.g. coro-noid(s), condyle(s), chin menton, and/or any other mandibular locations), one or more eye socket locations (e.g. lower orbit(s) and/or any other eye socket locations), auditory canal(s), one or more nose and/or skull locations (e.g. ANS, PNS, and/or any other nose and/or skull locations), one or more neck structures (e.g. upper cervical vertebras and/or any other neck structures), and/or any other anatomically relevant structures. The atlas anatomy data may typically be extracted from radiological or dental images, such as CT- images (e.g. CBCT-images), medical CT images, magnetic resonance images (MRI), and/or other radiological or dental images. Typically, these CT-images are 3D images, but the CT-images may also be 2D or 4D images. The atlas anatomy data may be extracted from a single subject or from multiple subjects. Thus, the atlas anatomy data may present a single subject anatomy or a mean anatomy of multiple subjects and/or it may comprise information on anatomy variations within the subjects (e.g. statistical atlas, probability atlas etc.). The images or volumes where the atlas anatomy data is extracted may also be a part of the atlas data 718. If multiple images are used, then a mean image/volume may be generated and may be included in the atlas data 718. Alternatively, the atlas anatomy data may also be created without the real images. Thus, the atlas anatomy data may also be artificial or generic.

There are many ways to represent the atlas anatomy data. The anatomical structures in the atlas data 718 may for example be represented as landmarks (e.g. 3D points), surfaces, pixels, voxels, or any known shapes (e.g. lines, planes, curves, etc.). For simplicity of the presentation, in next the representation of the anatomical data is limited for landmarks. However, it should be noted that this is only for simplicity of the disclosure, but the invention is not limited to this.

In addition to the atlas anatomy data and the possible image data, the atlas dental data may further comprise other geometrical information that is relevant for the X-ray dental imaging. The other geometrical information may for example comprise information related to a scan trajectory, a start position of the scan trajectory, an end position the scan trajectory, any other position of the scan trajectory, a full motion path, a dental arch, a sharp layer, information on exposure values along the scan trajectory, and/or any other similar information.

The atlas data 718 may be generated from data acquired from one or more atlas databases. The term "atlas" may also be called as model, mold, generic, artificial, template, arch, prototype, sample, framework and any other similar term. It also may be combined with terms such as dental, head, neck, skull, maxiofacial, jaw, maxilla, mandibular, head and neck, and/or any other similar terms. The atlas data 718 may be stored into a memory part 708 of the control system 106.

At step 430, the control system 106 may determine the dental arch data of the patient based on the positions of the detected plurality of anatomical structures determined at the step 420. For determining the dental arch data of the patient 300 the plurality of detected anatomical structures may comprise at least a plurality of dental arch related anatomical structures (e.g. a plurality of mandibular teeth, a plurality of maxillary teeth and/or any other dental arch related anatomical structures). However, the detected plurality of anatomical structures may further comprise any other one or more anatomical structures. The dental arch data represents an estimate of the position of the dental arch of the patient 300. As the positions of the detected plurality of anatomical structures are known (i.e. determined), the dental arch data of the patient 300 representing the estimate of the position of the dental arch of the patient 300 may be determined substantially accurately.

At the step 220, the control system 106 may for example control the parts of the dental X-ray imaging unit 102 to acquire the panoramic dental X-ray image data of the patient 300 according to the determined dental arch data. The control system 106 may for example control the gantry part 112 of the dental X-ray imaging unit 102 to scan the patient 300 according to a scan trajectory defined based on the determined dental arch data. The scan trajectory comprises a starting position of the panoramic scan and a motion path of the panoramic scan. The scan trajectory of the panoramic scan may be defined based on the dental arch data so that the imaging layer (i.e. a focal trough) corresponds to the dental arch of the patient 300. The imaging layer is a 3D-curved zone, where the patient's anatomy lying within this layer are sharp in the panoramic dental X-ray image and the other parts of the patient's anatomy are blurred in the panoramic dental X-ray image. This sharp layer is the narrowest at the anterior region (i.e. at the front teeth region). Thus, the scan trajectory of the panoramic scan may be defined so that at least the anterior region hits in the sharp layer, i.e. the imaging layer. According to an example, a patient specific scan trajectory may be defined based on the determined dental arch data. The patient specific scan trajectory enables that substantially the whole dental arch of the patient 300 hits in the sharp layer. In some cases, it may be possible that the dental X-ray imaging unit 102 cannot implement the patient specific scan trajectory and/or the patient specific scan trajectory cannot be defined. The scan trajectory may then be defined by selecting the most appropriate scan trajectory from among a plurality of predetermined scan trajectories that corresponds the best to the determined dental arch data. Each predetermined scan trajectory comprises the starting position of said predetermined scan trajectory and the motion path of said predetermined scan trajectory. For example, the control system 106 may compare the determined dental arch data and dental arches respective to the plurality of predetermined scan trajectories and select the scan trajectory whose respective dental arch corresponds the best to the determined dental arch data (e.g. the shape and/or the size of the dental arch). The controlling of the parts of the dental X-ray imaging unit 102 may comprise controlling the gantry part 112 to rotate according to the motion path of the panoramic scan around the rotation axis in order to acquire the panoramic dental X-ray image data of the patient 300. The motion path may for example be substantially an arched shaped path around the rotation axis. The rotation axis may be a mechanical rotation axis of the gantry part 112 or a virtual rotation axis of the gantry part 112. The controlling of the parts of the dental X-ray imaging unit 102 may also comprise controlling the gantry part 112 of the dental X-ray imaging unit 102 to move into the starting position of the panoramic scan, if the gantry part 112 is not already in the starting position of the panoramic scan. For example, to acquisition of the X-ray scout image 302 the gantry part 112 may be controlled to move into the starting position of the panoramic scan or at least very near to the starting position of the panoramic scan. Thus, if the gantry part 112 is already controlled to move into the starting position of the panoramic scan for the acquisition of the X-ray scout image 302, there is no need to control the gantry part 112 to move into the starting position of the panoramic scan for the acquisition the panoramic dental X-ray image data. To move the gantry part 112 into the starting position of the panoramic scan the rotation axis of the gantry part 112 may for example be placed in the starting position of the panoramic scan. Other techniques or alignments for the rotation axis may also be used as will be recognized by a person or ordinary skill in the art.

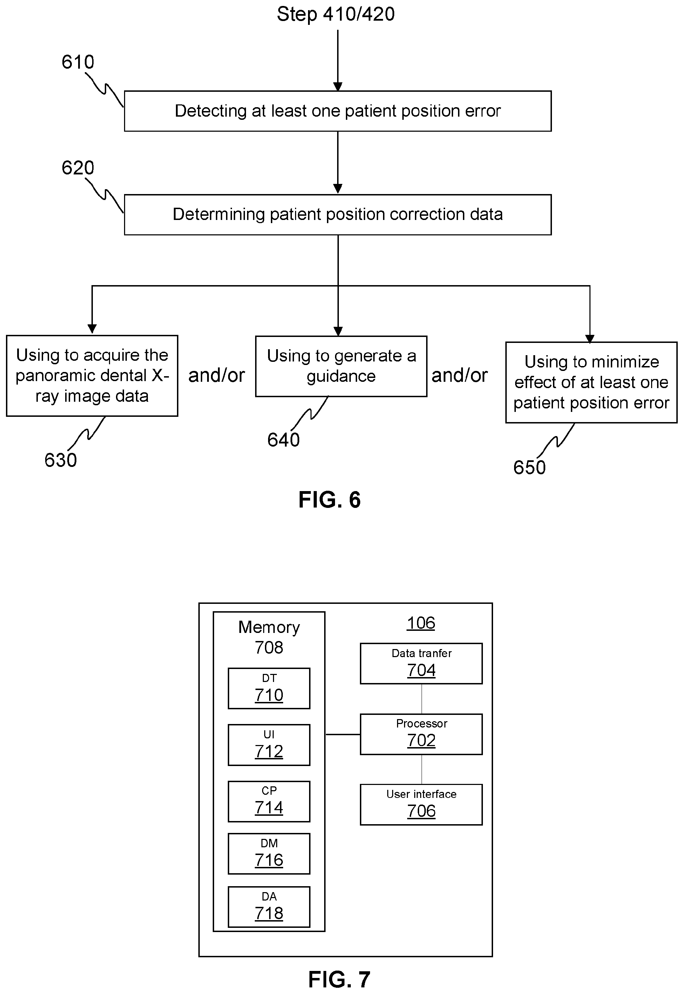

According to an example the plurality of anatomical structures detected from the X-ray scout image 302 may be used for correcting at least one patient position error. Depending on the patient position error, the one or more other structures detected from the X-ray scout image 302 may alternatively or in addition be used for correcting the patient position error in question. Figure 6 illustrates schematically an example of method steps of correcting the at least one patient position error. This enables detecting and correcting or at least minimizing different types of patient position errors, which in turn improves the quality of the resulting panoramic dental X-ray image.

At step 610, the control system 106 detects at least one patient position error based on the plurality of anatomical structures detected from the X-ray scout image 302. The control system 106 may further use the determined positions of the plurality of anatomical structures in the detection of the at least one patient position error. The at least one patient position error that may be detected based on the plurality of the anatomical structures may for example comprise at least one of the following: head tilt side-to-side, linear Anterior-Posterior (AP) movement, linear left-right (LR) movement, incorrect Frankfort-Horizontal (FH) line, slumped neck, uneven bite. The control system 106 may alternatively or in addition detect at least one patient position error based on the one or more other structures detected from the X-ray scout image 302. The control system 106 may further use the determined positions of the one or more other structures in the detection of the at least one patient position error. The at least one patient position error that may be detected based on the one or more other structures may for example comprise at least one of the following: shark-fin artefact, misplaced tongue. The detection of the shark-fin artefact and the misplaced tongue from the X-ray scout image 302 is discussed above.

The control system 106 may for example detect the uneven bite by detecting based on the plurality of anatomical structures detected from the X-ray scout image 302 that the mandibular and maxillary front teeth are not aligned. The control system 106 may for example detect the slumped neck by fitting a line through the upper cervical vertebras and compare said line to another line that is parallel with the principal beam from the X-ray source to the X-ray detector, when it is exposed directly behind the neck. In an optimal case (i.e. when there is no slumped neck), these lines intersect at 90 degrees angle. The smaller this angle is the more slumped the neck is, i.e. the larger the error caused by the slumped neck is. The control system 106 may for example detect out-of-plane rotations of the head of the patient 300 (e.g. the head tilt side-to-side, the linear AP movement, the linear LR movement, the incorrect FH line) by fitting lines and/or planes to the anatomical structures and by defining the angles between said lines and/or planes and respective reference lines and/or planes. There exist several options for anatomical structures to be used, which shapes (e.g. lines, planes, etc.) are fitted to the anatomical structures, and/or how the references are determined. Next some non-limiting examples are disclosed as non-limiting examples. A head nod (the incorrect FH line) may for example detected based on a line that passes through the cranial opening of the auditory canal and the lower orbit of the eye socket. The angle between said line and the horizontal plane should be zero, when the FH line is correct. If said angle is non-zero, the angle indicates the needed correction of the incorrect FH line. A head twist (i.e. the linear LR movement) may for example be detected based on an LR symmetry line. For example, nasion, ANS and chin menton may be used for determining the LR symmetry line. Further structures for determining the LR symmetry line may be defined by averaging symmetrical structures, e.g. any counter teeth on right and left side, any eye socket structures on right and left side, TMJs, etc. The LR symmetry line structures may be projected into the same coronal plane and a 2D line may be fitted to the LR symmetry line. In optimal case (i.e. when the head is straight) the angle between said 2D line and a vertical line in the imaging device coordinates is zero. If said angle is non-zero, the angle indicates the needed correction of the linear LR movement).

If the selected scan trajectory is used instead of the patient specific scan trajectory, the at least one patient position error may comprise at least one scan trajectory deviation. The selected scan trajectory and the dental arch respective to the selected scan trajectory deviates from the determined dental arch data. The at least one scan trajectory deviation may for example comprise at least one of the following: linear deviation in the horizontal plane, deviation of an angle of rotation in the horizontal plane. In the linear deviation in the horizontal plane, the selected scan trajectory and the dental arch respective to the selected scan trajectory deviates from the determined dental arch data of the patient 300 in the horizontal plane. The control system 106 may for example determine the linear deviation between the locations of the dental arch respective to the selected scan trajectory and the determined dental arch data of the patient 300. As the shape and the size of the dental arches deviate from each other, the dental arches may be fitted accurately only partially. As the sharp layer is the narrowest at the anterior region (i.e. at the front teeth region) as discussed above, the fitting of the dental arch respective to the selected scan trajectory to the determined dental arch data of the patient 300 may preferably be made at this region, e.g. based on the locations of the front teeth. For example, the control system 106 may determine position vectors of the front teeth (e.g. center point of the front teeth) in the horizontal plane for the front teeth of the dental arch data of the patient 300 and for the dental arch respective to the selected scan trajectory. The difference of the position vectors indicates the linear deviation between the dental arch data of the patient 300 and for the dental arch respective to the selected scan trajectory. The deviation of the angle of rotation in the horizontal plane is caused by turning of the head of the patient in the horizontal plane (i.e. a yaw rotation of the head of the patient 300). The control system 106 may for example determine the deviation of the angle of rotation in the horizontal plane by utilizing PA-axes. A line parallel to the PA-axis of the patient 300 may for example be determined based on the LR symmetry line. For example, nasion, ANS and chin menton may be used for determining the LR symmetry line. Further structures for determining the LR symmetry line may be defined by averaging symmetrical structures, e.g. any counter teeth on right and left side, any eye socket structures on right and left side, TMJs, etc. By projecting the LR symmetry line structures into the horizontal plane, a line may be fitted to these LR symmetry line structures. Said line defines the line parallel to the PA axis of the patient 300. A line parallel to the PA-axis of the selected scan trajectory is known or may be determined similarly as the line parallel to the PA-axis of the patient 300 described above. The control system 106 may then determine the angle between the line parallel to the PA-axis of the patient 300 and the line parallel to the PA-axis of the selected scan trajectory. Said angle corresponds to the angle of rotation in the horizontal plane.

At step 620, the control system 106 determines patient position correction data for correcting the at least one patient position error. The patient position correction data represents the correction needed to correct the at least one patient position error. The patient position correction data may for example be determined substantially in pursuance of detecting the at least one patient position error.

Depending on the detected at least one patient position error the control system 106 may perform one or more different type patient position error correction actions 630-650 based on the determined patient position correction data.

The control system 106 may use 630 the patient position correction data in the controlling of the parts of the dental X-ray imaging unit 102 to acquire the panoramic dental X-ray image data of the patient 300. According to an example, if the detected at least one patient position error comprises the linear AP movement, the control system 106 may move the gantry part 112 in AP direction until the liner AP movement is compensated according to determined patient position correction data. To move the gantry part 112 the control system may control the at least one linear motor to move the gantry part 112 in the AP direction. According to another example, if the detected at least one patient position error comprises the linear LR movement, the control system 106 may move the gantry part 112 by pivoting until the liner LR movement is compensated according to determined patient position correction data. To pivot the gantry part 112 the control system 106 may control the pivot motor to pivot the upper shelf around the supporting column 103. According to yet another example, if the detected at least one patient position error comprises incorrect Frankfort-Horizontal line, the control system 106 may adjust the height of the parts of the dental X-ray imaging unit 102 until the incorrect Frankfort-Horizontal line is compensated according to determined patient position correction data. To adjust the height of the parts of the dental X-ray imaging unit 102 the control system 106 may control the guide motor to move the carriage part 101 up or down along the supporting column 103 in the height direction Z. According to yet another example, if the detected at least one patient position error comprises slumped neck, the control system 106 may adjust the height of the parts of the dental X-ray imaging unit 102 until the slumped neck is compensated according to determined patient position correction data (i.e. the slumped neck is outside the image). According yet another example, if the detected at least one patient position error comprises the head tilt side-to-side, the control system 106 may adjust the head support part 126 until the head tilt is compensated according to the determined patient position correction data. According to yet another example, if the detected at least one patient position error comprises shark-fin artefact, the control system 106 may adjust the height of the parts of the dental X-ray imaging unit 102 until the shark-fin artefact is compensated according to determined patient position correction data (i.e. the shark-fin artefact is outside the panoramic FOV). According to yet another example, if the detected at least one patient position error comprises at least one scan trajectory deviation (being the linear deviation in the horizontal plane and/or the deviation of the angle of rotation in the horizontal plane), the control system 106 may adjust the head support part 126 until the scan trajectory deviation is compensated according to the determined patient position correction data. If the detected at least one patient position error comprises at least one scan trajectory deviation being the linear deviation in the horizontal plane, the control system 106 may further adjust the starting position of the panoramic scan until the scan trajectory deviation is compensated according to the determined patient position correction data. If the detected at least one patient position error comprises at least one scan trajectory deviation being the deviation of the angle of rotation in the horizontal plane, the control system 106 may further adjust the angle of the starting position of the panoramic scan and/or pivot the axes of the X-ray imaging unit 102 (e.g. PA axis of the X-ray imaging unit 102) until the scan trajectory deviation is compensated according to the determined patient position correction data

Alternatively or in addition, the control system 106 may generate 640 a guidance to the patient 300 and/or to an operator of the dental X-ray imaging system 100 based on the patient position correction data. For example, the guidance may be provided via one or more user interface devices e.g. via a display device and/or a loudspeaker device. The one or more user interface device may comprise the user interface device 130 and/or one or more other user interface devices. The guidance is performed during the imaging process. Depending on the type of the at least one patient position error, the action to compensate the at least one patient position error in response to the guidance may be performed during the imaging process or the imaging process may be interrupted for performing of the action to compensate the at least one patient position error. According to an example, if the detected at least one patient position error comprises the slumped neck, the control system 106 may generate a guidance comprising an instruction for the patient 300 to step forward to compensate the slumped neck. According to another example, if the detected at least one patient position error comprises the shark-fin artefact, the control system 106 may generate a guidance comprising an instruction to remove any foreign objects (e.g. accessories of the patient 300) and/or adjust a protective gear(s) (e.g. an apron) to compensate (e.g. remove) the shark-fin artefact. According to yet another example, if the detected at least one patient position error comprises misplaced tongue, the control system 106 may generate a guidance comprising an instruction for the patient 300 to correct the location of the tongue to compensate the misplaced tongue, for example during the imaging process. The correction of the location of the tongue to compensate the misplaced tongue may for example performed during the imaging process. According to yet another example, if the detected at least one patient position error comprises uneven bite, the control system 106 may generate a guidance comprising an instruction for the patient 300 to correct the bite on the bite stick to compensate the uneven bite. The correction of the bite on the stick to compensate the uneven bite may for example performed during the imaging process. According to yet another example, if the detected at least one patient position error comprises at least one scan trajectory deviation (being the linear deviation in the horizontal plane and/or the deviation of the angle of rotation in the horizontal plane), the control system 106 may generate a guidance comprising an instruction for the patient 300 to correct the posture of the head to compensate the scan trajectory deviation.

Alternatively or in addition, the control system 106 may use 650 the patient position correction data to minimize the effect of at least one patient position error. The minimizing of the effect of the at least one patient position error may for example comprise at least one of the following: adjusting exposure parameters for the panoramic scan, postprocessing the projection images from which the dental X-ray image is formed by reconstructing the X-ray image data, postprocessing reconstruction parameters, postprocessing the dental X-ray image reconstructed from the obtained X-ray image data. According to an example, if the detected at least one patient position error comprises slumped neck, the control system 106 may adjust the exposure (i.e. radiation) parameters so that the effect of the slumped neck is minimized according to determined patient position correction data. For example, the exposure parameters may be adjusted so that the exposure is increased in the directions in which the patient 300 is exposed through the slumped neck. According to another example, if the detected at least one patient position error comprises misplaced tongue, the control system 106 may adjust the exposure (i.e. radiation) parameters so that the effect of the misplaced tongue is minimized according to determined patient position correction data. The misplaced tongue (e.g. when the tongue is not in the palate) may for example cause an air cap in the oral cavity above the tongue, which in turn may cause that too much exposure ends up to the X-ray detector. In this case, the exposure parameters may be adjusted so that the exposure is reduced. One or more other exposure parameters (e.g. the size of the collimation area and/or size of the second part of the active area of the X-ray detector) may alternatively or in addition be adjusted to minimize the effect of the at least one patient position error according to determined patient position correction data. According to yet another example, if the detected at least one patient position error comprises slumped neck, the control system 106 may postprocess the projection images for example by increasing noise suppression in the projection images that have been acquired behind the neck so that the effect of the slumped neck is minimized according to determined patient position correction data. According to yet another example, if the detected at least one patient position error comprises uneven bite, the control system 106 may postprocess the reconstruction parameters so that the effect of the uneven bite is minimized according to determined patient position correction data. According to yet another example, if the detected at least one patient position error comprises the head tilt side-to-side, the control system 106 may postprocess the dental X-ray image reconstructed from the obtained X-ray image data so that the effect of the head tilt side-to-side is minimized according to determined patient position correction data.

Figure 7 illustrates a schematic example of the control system 106 of the dental X-ray imaging system 100. The control system 106 may comprise a processor part 702, a data transfer part 704, a user interface part 706, and a memory part 708. The processor part 702 is configured to perform user and/or computer program (software) initiated instructions, and to process data. The processor part 702 may comprise at least one processor. The memory part 708 is configured to store and maintain data. The data may be instructions, computer programs, the at least one trained detection model 716, and any data files. The memory part 708 may comprise at least one memory. The memory part 708 may further comprise at least a data transfer application 710 in order to control the data transfer part 704, a user interface application 712 in order to control the UI part 706, and a computer program (code) 714 in order to control the operations of the control system 106. The memory part 708 and the computer program 714, together with the processor part 702, may cause the control system 106 at least to implement one or more method steps and/or operations of the control system 106 as described above. The data transfer part 704 may be configured to send control commands other units, e.g. the dental X-ray imaging unit 102. In addition, the data transfer part 704 may receive data from other units, e.g. the dental X-ray imaging unit 102, a database(s) and/or any other external units. The user interface (UI) part 706 may be configured to input control commands, to receive information and/or instructions, and to display information. The UI part 706 may comprise one or more user interface devices 130, e.g. a display, a screen, a touchscreen, at least one function key, a keyboard, a wired or wireless remote controller, and/or any other user input and/or output device. The computer program 714 may be a computer program product that may be comprised in a tangible, non-volatile (non-transitory) computer-readable medium bearing the computer program code 714 embodied therein for use with a computer, e.g. the control system 106.

The specific examples provided in the description given above should not be construed as limiting the applicability and/or the interpretation of the appended claims. Lists and groups of examples provided in the description given above are not exhaustive unless otherwise explicitly stated.

## Claims

1. A dental X-ray imaging system (100) for dental X-ray imaging of a patient (300), the system (100) comprises:
a dental X-ray imaging unit (102) comprising:
an X-ray source part (114) for emitting an X-ray beam,
an X-ray imaging detector part (116) comprising one X-ray detector for receiving the X-ray beam from the X-ray source part (114), and
a gantry part (112) comprising the X-ray source part (114) and the X-ray imaging detector part (116); and
a control system (106) configured to:
control the parts of the dental X-ray imaging unit (102) to acquire an X-ray scout image (302) of the patient (300) by using a first part of an active area of the X-ray detector, and
control the parts of the dental X-ray imaging unit (102) to acquire panoramic dental X-ray image data of the patient (300) by using a second part of the active area of the X-ray detector being respective to the panoramic imaging,
wherein the first part of the active area is larger than the second part of the active area.

2. The dental X-ray imaging system (100) according to claim 1, wherein the first part of the active area of the X-ray detector comprises the entire active area of the X-ray detector.

3. The dental X-ray imaging system (100) according to any of the preceding claims, wherein the control of the parts of the dental X-ray imaging unit (100) to acquire the X-ray scout image (302) comprises controlling a collimator of the X-ray source part (114) to collimate the X-ray beam into a cone beam.

4. The dental X-ray imaging system (100) according to any of the preceding claims, wherein the control of the parts of the dental X-ray imaging unit (102) to acquire the panoramic dental X-ray image data comprises controlling the collimator of the X-ray source part (114) to collimate the X-ray beam into a narrow beam.

5. The dental X-ray imaging system (100) according to any of the preceding claims, wherein the control system (106) is configured to determine dental arch data of the patient based on the X-ray scout image (302).

6. The dental X-ray imaging system (100) according to claim 5, wherein the control system (106) is further configured to control the parts of the dental X-ray imaging unit (102) to acquire the panoramic dental X-ray image data of the patient according to the determined dental arch data.

7. The dental X-ray imaging system (100) according to any of claims 5 or 6, wherein the determining the dental arch data comprises that the control system (106) is configured to:
detect a plurality of anatomical structures of the patient (300) from the X-ray scout image (302),
determine positions of the plurality of anatomical structures of the patient (300), and
determine dental arch data of the patient (300) based on the determined positions of the detected plurality of anatomical structures of the patient (300).

8. The dental X-ray imaging system (100) according to claim 7, wherein the control system (106) is configured to apply at least one trained detection model (716) to detect the plurality of anatomical structures of the patient (300) from the X-ray scout image (302).

9. The dental X-ray imaging system (100) according to claim 8, wherein the at least one trained detection model (716) is a machine learning (ML) model or an artificial intelligence (AI) model.

10. The dental X-ray imaging system (100) according to any of claims 7 to 9, wherein the control system (106) is configured to determine the positions of the plurality of anatomical structures of the patient (300) by utilizing atlas data (718).

11. The dental X-ray imaging system (100) according to any of claims 7 to 10, wherein the control system (106) is configured to:
detect at least one patient position error based on the plurality of anatomical structures of the patient (300), and
determine patient position correction data for correcting the at least one patient position error.

12. The dental X-ray imaging system (100) according to claim 11, wherein the control system (106) is configured to perform at least one of the following:
use the patient position correction data in the controlling of the parts of the dental X-ray imaging unit (102) to acquire the panoramic dental X-ray image data of the patient (300),
generate a guidance to the patient (300) and/or to an operator of the dental X-ray imaging system (100) based on the patient position correction data,
use the patient position correction data to minimize the effect of at least one patient position error.

13. A method for a panoramic dental X-ray imaging of a patient (300), the method is performed by an X-ray dental imaging system (100) according to any of the preceding claims, wherein the method comprises:
controlling (210) the parts of the dental X-ray imaging unit (102) to acquire an X-ray scout image (302) of the patient (300) by using a first part of an active area of the X-ray detector, and
controlling (220) the parts of the dental X-ray imaging unit (102) to acquire panoramic dental X-ray image data of the patient (300) by using a second part of the active area of the X-ray detector being respective to the panoramic imaging,
wherein the first part of the active area is larger than the second part of the active area.

14. A computer program (714) comprising instructions which, when the program is executed by an X-ray dental imaging system (100) according to any of claims 1 to 12, cause the X-ray dental imaging system (100) to carry out the method according to claim 13.

15. A tangible non-volatile computer-readable medium comprising instructions which, when executed by an X-ray dental imaging system (100) according to any of claims 1 to 12, cause the X-ray dental imaging system (100) to carry out the method according to claim 13.
